# NEUE EUROPÄISCHE PATENTSCHRIFT

(11) **EP 0 682 505 B2**
(45) Veröffentlichungstag und Bekanntmachung des Hinweises auf die Entscheidung über den Einspruch: **12.11.2003**
(45) Hinweis auf die Patenterteilung: 15.10.1997
(21) Anmeldenummer: 94905682.4
(22) Anmeldetag: 22.01.1994
(51) Int. Cl.: A61F 2/06

(54) **STENT**
STENT
EXTENSEUR

(30) Priorität: 04.02.1993 DE 4303181
(43) Veröffentlichungstag der Anmeldung: 22.11.1995
(73) Patentinhaber: Angiomed GmbH & Co. Medizintechnik KG, 76227 Karlsruhe (DE)
(72) Erfinder: SCHNEPP-PESCH, Wolfram, D-76227 Karlsruhe (DE); LINDENBERG, Josef, D-76227 Karlsruhe (DE)
(74) Vertreter: Schweighart, Peter
(86) Internationale Anmeldenummer: EP9400168
(87) Internationale Veröffentlichungsnummer: WO94017754

(56) Entgegenhaltungen:
- EP-A- 0 540 290
- EP-B- 0 335 341
- WO-A-92/06734
- DE-B- 1 766 921
- US-A- 4 390 599
- US-A- 5 104 404
- US-A- 5 135 536

## Beschreibung

Die Erfindung betrifft einen Stent nach dem Oberbegriff des Anspruchs 1.

Derartige in einen Körperhohlraum, ein Gefäß oder dergleichen einbringbare Stents oder implantierbare Katheter können aus Kunststoff oder aus inertem Metall, wie Stahl oder Nickel-Titan-Legierungen, bestehen. Solche Stents werden insbesondere auch als endovaskuläre bzw. endoluminale Stents bzw. Endoprothesen bezeichnet. Die Stents werden beispielsweise zur Erweiterung des Harnleiters im Prostatabereich bei benigner Prostata-Hyperplasie (BPH) oder aber auch in verkalkten Blutgefäßen zur Erweiterung und Offenhaltung derselben eingesetzt. Die Stents weisen Materialbereiche und Zwischenräume zwischen diesen auf. Hierdurch kann ein Umwachsen des Stents durch das Wandungsgewebe des offengehaltenen Organs erfolgen. Stents können spiralig oder in Form einer schraubenförmig gewundenen Wendel ausgebildet sein; sie können aus gewebtem oder gestricktem Draht- oder Kunststoffmaterial bestehen.

Ein Problem bei derartigen Stents ist ihre beschränkte Biegefähigkeit, insbesondere beim Einführen durch enge Organe, wie Blutgefäße, an den Ort, an dem eine Erweiterung vorgenommen werden kann. Es besteht die Gefahr, daß bei einer Biegung des Stents durch Einwirken achssenkrechter Kräfte der Stent in der Mitte praktisch einknickt, indem sein Querschnittsbereich in Richtung der einwirkenden Kräfte reduziert, senkrecht hierzu und zu ihrer Achsrichtung aber erweitert wird. Dies kann das Einführen erschweren und außerdem zu Beschädigungen des umgebenden Gewebes führen, insbesondere wenn der Stent in einem Biegungsbereich des Gefäßes oder dergleichen eingesetzt werden soll. Die Stents sind relativ steif und unflexibel.

Aus der US-A-5 135 536 und der US-A-5 104 404 sind jeweils Stents bekannt, die mehrere in Achsrichtung hintereinander angeordnete, sich über einen Umfang erstreckende Mäanderbahnen aufweisen, wobei einander in Achsrichtung zugewandte Bereiche durch Verbindungsabschnitte miteinander verbunden, jedoch weitere in Umfangsrichtung einander zugewandte Bereiche nicht miteinander verbunden sind.

Der Erfindung liegt daher die Aufgabe zugrunde, einen Stent zu schaffen, der eine hohe Biegeflexibilität bei achssenkrecht einwirkenden Kräften aufweist, dabei sämtlichen Biegungen eines Hohlraums im Körper ohne Probleme folgen kann und insbesondere keinen Deformationen seiner Kontur unterliegt, insbesondere bei Biegungen keine Querschnittsänderungen erleidet.

Erfindungsgemäß wird die genannte Aufgabe durch einen gattungsgemäßen Stent mit den kennzeichenden Merkmalen des Anspruchs 1 gelöst.

Dadurch, daß bei einem derartigen Stent mit mehreren in Achsrichtung hintereinander angeordneten, über den Umfang hin mäanderförmig geführten Materialbahnen einander zugewandte bzw. aufeinander zu gerichtete benachbarte Bereiche zweier benachbarter Mäanderbahnen nicht in jedem Falle miteinander verbunden sind, sondern zwischen miteinander verbundenen derartigen Bereichen in Umfangsrichtung hin mindestens jeweils zwei nicht verbundene Bereiche vorgesehen sind, wird eine höhere Flexibilität erreicht, als es bei einem Stent der Fall wäre, bei dem sämtliche einander zugewandten, benachbarten Bereiche zweier benachbarter Mäanderbahnen fest miteinander verbunden sind. Hierdurch wird nicht nur eine höhere Flexibilität erreicht, sondern es wird insbesondere auch erreicht, daß bei Biegungen unter Einwirkung achssenkrechter Kräfte keine Querschnittsdeformation erfolgt.

Ein wesentlicher Vorteil der Erfindung liegt darin, daß eine hohe Biegefähigkeit erreicht wird ohne mehrlagige Materialkreuzungspunkte, wie dies bei Gestrikken, Geflechten usw. der Fall ist. Dadurch, daß solche Materialkreuzungspunkte fehlen, erfolgt ein besseres Einwachsen des erfindungsgemäßen Stents ins Gewebe. Weiter wird hierdurch die Gefahr des Auftretens von Thrombosen, insbesondere im vaskulären Bereich, wesentlich reduziert bzw. praktisch ausgeschlossen.

In bevorzugter Ausgestaltung kann dabei vorgesehen sein, daß die Verbindungsabschnitte in axialer Richtung aufeinanderfolgender Mäanderbahnen in Umfangsrichtung versetzt zueinander angeordnet sind, wobei insbesondere die Verbindungsabschnitte um eine halbe Mäanderperiode in Umfangsrichtung versetzt angeordnet sind. Hierdurch wird die erwünschte Axialfestigkeit beibehalten bzw. erreicht.

Die Mäanderbahnen können in vielfältiger Weise ausgebildet sein. So sehen bevorzugte Ausgestaltungen vor, daß die Mäanderbahnen zickzackförmig (mit Spitzen) ausgebildet sind, daß die Mäanderbahnen sinusförmig ausgebildet sind oder auch daß die Mäanderbahnen ovalförmig ausgebildet sind. Weitere bevorzugte Ausgestaltungen der Erfindung sehen vor, daß einander zugewandte Bereiche der Mäanderbahnen in Achsrichtung fluchten und/oder daß die Breite der Verbindungsbereiche in Umfangsrichtung nicht größer als die Breite der Schenkel der Mäanderbahnen ist.

Der Stent ist vorzugsweise selbstexpandierend und besteht aus einem Material mit Gedächtniseigenschaften (Memory-Metall); im Tieftemperaturzustand (weit unter Körpertemperatur) liegen die einzelnen Mäanderschenkel aneinander an, im vorgeprägten Hochtemperaturzustand (unterhalb, aber näher der Körpertemperatur) ist der Stent radial aufgeweitet.

Weitere Vorteile und Merkmale der Erfindung ergeben sich aus den Ansprüchen und aus der nachfolgenden Beschreibung, in der der erfindungsgemäße Stent unter Bezugnahme auf die Zeichnung im einzelnen erläutert ist. Dabei zeigt:
- Figur 1: eine bevorzugte Ausgestaltung des erfindungsgemäßen Stents in seiner Tieftemperatur- oder Einbringkonfiguration;
- Figur 2: den Stent der Figur 1 in seiner Hochtemperatur- oder Positionierkonfiguration;
- Figur 3: eine schematische Darstellung eines in Längsrichtung an seinen Schweißstellen aufgetrennten und flach ausgelegten Stents zur besseren Verdeutlichung der Verbindung der in Achsrichtung hintereinander angeordneten, zickzackförmigen Mäanderbahnen; und
- Figur 4: ein zur Herstellung eines erfindungsgemäßen Stents vorgesehenes Schlitzblech.

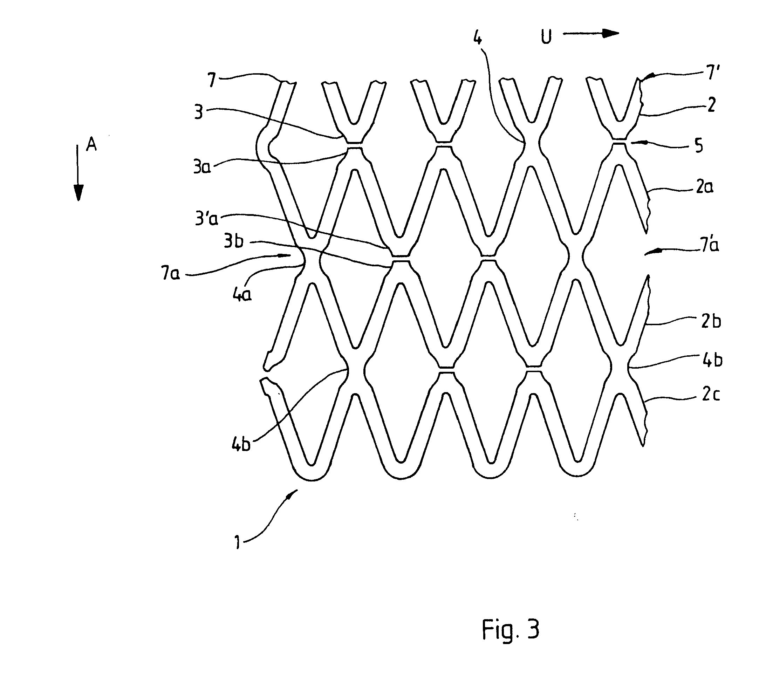

Der erfindungsgemäße Stent 1 weist in der dargestellten Ausführungsform eine grundsätzlich zylindrische Form auf, wobei die Außenkontur des Stents in der Figur 2 durch gestrichelte Linien S angedeutet ist.

Statt einer zylindrischen Ausgestaltung kann der Stent 1 auch konus- oder doppelkonusförmige oder aber kegelstumpfförmige sowie andere Konturen aufweisen. Auf jeden Fall weist er eine Symmetrieachse A auf, die die Achsrichtung bestimmt. Weiterhin ist die Umfangsrichtung durch den Pfeil U angedeutet.

Der erfindungsgemäße Stent 1 besteht, wie insbesondere aus den Figuren 2 und 3 deutlich wird, aus einer Reihe von in Achsrichtung A hintereinander angeordneten Mäanderbahnen 2, 2a, 2b. in Umfangsrichtung sind die Mäanderbahnen 2, 2a, 2b derart angeordnet, daß jeweils einander zugewandte, benachbarte Spitzenbereiche 3, 3a bzw. 3'a, 3b von jeweils nebeneinander angeordneten Mäanderbahnen 2, 2a, 2b in Achsrichtung fluchten.

Den Figuren 2 und 3 ist ebenfalls deutlich zu entnehmen, daß nicht sämtliche einander zugewandten, benachbarten Spitzenbereiche 3, 3a, 3'a, 3b der Mäanderbahnen 2, 2a, 2b durch Verbindungsbereiche 4, 4a, 4b, 4c, 4d miteinander verbunden sind, sondern zwischen derartigen Verbindungsbereichen 4 bis 4d zweier benachbarter Mäanderbahnen 2, 2a jeweils in Umfangrichtung mehrere Lücken 5, 5', 5a, 5b, 5b' usw. angeordnet sind. Hierdurch wird eine hohe Flexibilität des erfindungsgemäßen Stents erreicht. Es wird insbesondere erreicht, daß der Stent 1 bei Biegung senkrecht zu seiner Längsachse A und damit Biegung der Längsachse A selbst nicht im Mittelbereich derart einknickt, daß er seine im Querschnitt im wesentlichen kreisförmige Kontur verliert und in Einwirkrichtung der Kräfte in der Mitte flachgedrückt und senkrecht zur Einwirkungsrichtung der Kräfte etwa in der Mitte seiner Längserstreckung verbreitert wird, wie dies bei herkömmlichen Stents der Fall ist, bei denen sämtliche einander zugewandten, benachbarten Spitzenbereiche 3, 3a etc. nebeneinander verlaufender Mäanderwindungen durch Verbindungsbereiche 4, 4a etc. fest verbunden sind.

Die Verbindungsbereiche 4, 4a ... sind einstückig mit den sonstigen Teilen des Stents, insbesondere den Mäanderbahnen 2, 2a ... und deren jeweils einander benachbarten Bereichen 3, 3a ausgebildet.

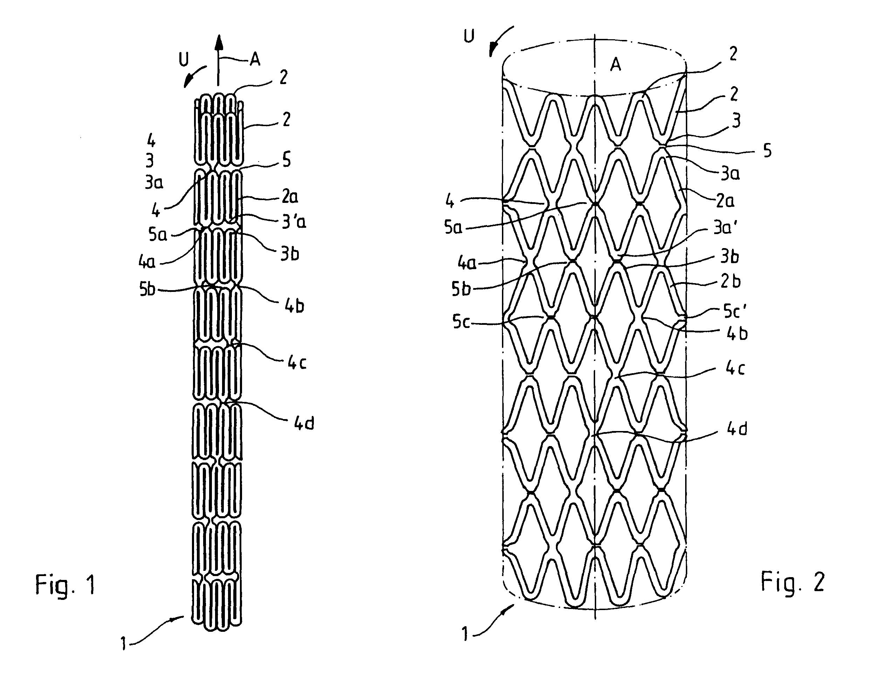

Der Figur 1 ist zu entnehmen, daß die zwischen den Schenkeln der Mäanderbahnen 2, 2a etc. in der Hochtemperaturstellung ausgebildeten, im wesentlichen rautenförmigen Freiräume in der Niedertemperaturstellung sich zu Schlitzen verjüngen und die Schenkel der Mäanderbahnen 2 ... im wesentlichen parallel zueinander verlaufen.

Der Figur 3 ist darüber hinaus zu entnehmen, daß die Stärke der Verbindungsbereiche 4, 4a, 4b, 4c in Umfangsrichtung nicht größer ist als die Stärke der einzelnen Schenkel der Mäanderbahnen 2, 2a, ...

Die Bereiche 7, 7' bzw. 7a, 7a' sind Schweißbereiche, die in geschlossener Stellung des in Figur 3 dargestellten Stents durch Schweißverbindungen miteinander verbunden sind.

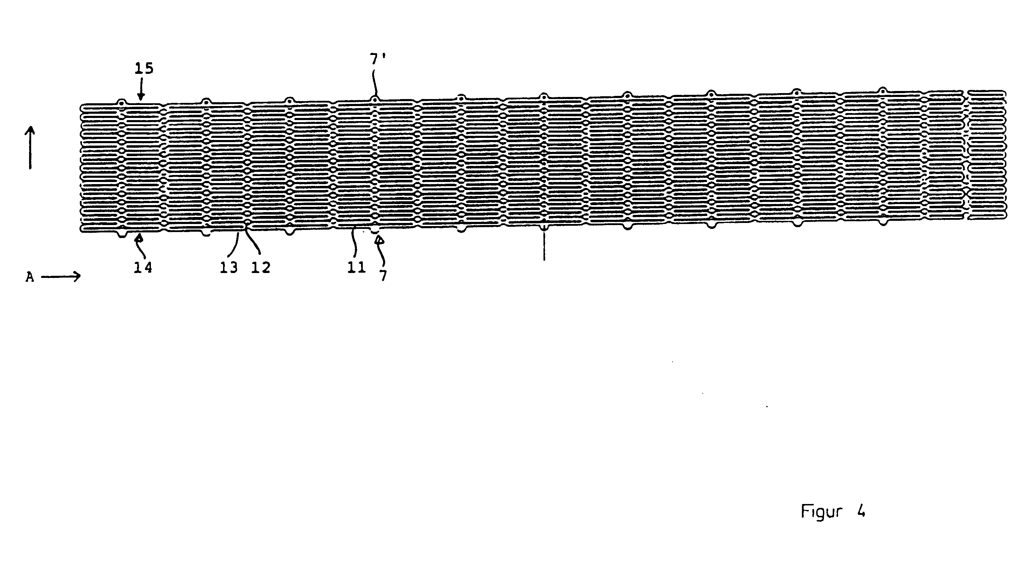

Die Figur 4 zeigt ein schon geschlitztes Blech, aus dem ein erfindungsgemäßer Stent hergestellt wird.

Der erfindungsgemäße Stent besteht aus einer Nikkel-Titan-Legierung, wie aus Nitinol®. In einem flächigen Blech werden die Durchbrüche oder Schlitze 11, wie sie in der Figur 4 dargestellt sind, derart erzeugt, daß in Umfangsrichtung U benachbarte Schlitze jeweils etwa um die Hälfte ihrer Länge in Achsrichtung A versetzt sind. Im Mittelbereich jedes Schlitzes 11 ist dieser mit einer Erweiterung 12 versehen, so daß das die Erweiterung 12 in Umfangsrichtung begrenzende Material etwa auf die Breite der zwischen den Schlitzen selbst verbliebenen Materialbestände reduziert wird. Die Abschnitte 13 bilden später, wenn sie stehengelassen werden, die Verbindungsabschnitte 4, 4a etc., oder es werden in ihren Bereichen, wenn die Abschnitte 13 entfernt werden, die Frei- oder Zwischenräume 5, 5a etc. geschaffen.

Nach dem Herstellen des Bleches in der in Figur 4 ersichtlichen Form werden die Abschnitte 13 zunächst sämtlich stehengelassen. Lediglich links wurde in der Figur 4 angedeutet, wie später, d.h. nach Herstellen des Stents, wie er in den Figuren 1 und 2 dargestellt ist, hier die Trennungen zur Schaffung der Zwischenräume 5 erzeugt werden.

Das in der Figur 4 dargestellte Blech wird zu einem Zylinder gebogen, so daß die beiden Ränder 14, 15 sich berühren. Es werden dann an den Schweißpunkten 7, 7' die Verschweißungen vorgenommen, wodurch zunächst ein Stent in seiner Tieftemperaturstellung entsprechend der Figur 1 entsteht. Anschließend erfolgt eine Wärmebehandlung, um dem so beschaffenen Stent seine Gedächtniseigenschaften (Memory-Eigenschaften) zu verleihen, so daß er nach Temperaturerhöhung über eine vorgegebene Umgebungstemperatur, . die unterhalb der Körpertemperatur des menschlichen Körpers liegt, sich in seine Hochtemperaturstellung entsprechend der Figur 2 aufweiten kann.

Nachdem der Stent derart hergestellt und wärmebehandelt wurde, werden dann Brücken 13 in der gewünschten Weise entfernt, so daß die Verbindungsbereiche oder Stege 4, 4a etc. bzw. Freiräume 5, 5', 5a etc. gebildet werden, wie dies oben beschrieben wurde. In der Figur 3 sind zwischen zwei in Umfangsrichtung aufeinanderfolgenden Verbindungsbereichen oder Stegen 4, 4a jeweils zwei Freiräume 5 benachbarter, einander zugewandter Bereiche 3, 3a der Mäanderwindungen 2, 2a geschaffen worden. Die Abstände zwischen den Verbindungsbereichen 4 in Umfangsrichtung können auch größer gewählt werden; in der Regel sollten mindestens zwei Freibereiche 5 zwischen zwei in Umfangsrichtung aufeinanderfolgenden Stegen 4 vorgesehen sein.

Durch die Erfindung wird insgesamt ein hochflexibler Stent geschaffen, der sämtlichen Biegungen ohne jegliche Beeinträchtigung folgen kann.

## Patentansprüche

1. Stent mit mehreren in Achsrichtung hintereinander angeordneten, sich über seinen Umfang (U) erstreckenden Mäanderbahnen (2, 2a, 2b, 2c), wobei einander in Achsrichtung (A) zugewandte Bereiche durch Verbindungsabschnitte (4, 4a, 4b, 4c) miteinander verbunden, jedoch weitere in Umfangsrichtung zwischen diesen angeordnete, in Achsrichtung einander zugewandte Bereiche (3, 3a; 3a', 3b) nicht miteinander verbunden sind, **dadurch gekennzeichnet, daß** der Stent (1) aus Flachblech einer Formgedächtnislegierung mit einer Tieftemperaturkonfiguration, bei der die Mäanderschenkel der Mäanderbahnen (2, 2a, 2b, 2c) aneinander anliegen, und einer radial aufgeweiteten Hochtemperaturkonfiguration besteht und daß zwischen jeweils zwei Verbindungen (4, 4a, 4b, 4c) in derselben Mäanderbahn (2, 2a, 2b, 2c) mindestens jeweils zwei nicht miteinander verbundene Bereiche (3, 3a; 3a', 3b) liegen.

2. Stent nach Anspruch 1, **dadurch gekennzeichnet, daß** die Verbindungsabschnitte (4, 4a, 4b, 4c) in axialer Richtung aufeinanderfolgender Mäanderbahnen (2, 2a, 2b, 2c) in Umfangsrichtung (U) versetzt zueinander angeordnet sind.

3. Stent nach Anspruch 2, **dadurch gekennzeichnet, daß** die Verbindungsabschnitte (4, 4a, 4b, 4c) um eine halbe Mäanderperiode in Umfangsrichtung (U) versetzt angeordnet sind.

4. Stent nach einem der Ansprüche 1 bis 3, **dadurch gekennzeichnet, daß** die Mäanderbahnen (2, 2a, 2b, 2c) zickzackförmig (mit Spitzen) ausgebildet sind.

5. Stent nach einem der Ansprüche 1 bis 3, **dadurch gekennzeichnet, daß** die Mäanderbahnen sinusförmig ausgebildet sind.

6. Stent nach einem der vorangehenden Ansprüche, **dadurch gekennzeichnet, daß** die Mäanderbahnen ovalförmig ausgebildet sind.

7. Stent nach einem der vorangehenden Ansprüche, **dadurch gekennzeichnet, daß** einander zugewandte Bereiche (3, 3a, 3'a, 3b) der Mäanderbahnen (2, 2a, 2b, 2c) in Achsrichtung (A) fluchten.

8. Stent nach einem der vorangehenden Ansprüche, **dadurch gekennzeichnet, daß** die Breite der Verbindungsbereiche (4, 4a, 4b, 4c) in Umfangsrichtung (U) nicht größer als die Breite der Schenkel der Mäanderbahnen (2, 2a, 2b, 2c) ist.

## Claims

1. Stent having several, axially successively arranged meandering paths (2, 2a, 2b, 2c) extending over its circumference (U), facing areas in the axial direction (A) being interconnected by connecting portions (4, 4a, 4b, 4c), but further areas (3, 3a, 3a', 3b) facing one another in the axial direction and circumferentially located between the first-mentioned areas are not interconnected, **characterized in that** the stent (1) comprises a flat, shape memory alloy sheet with a low temperature configuration, in which the meandering legs of the meandering paths (2, 2a, 2b, 2c) engage with one another and a radially expanded high temperature configuration and that between in each case two connections (4, 4a, 4b, 4c) in the same meandering path (2, 2a, 2b, 2c) there are in each case at least two not interconnected area (3, 3a, 3a', 3b).

2. Stent according to claim 1, **characterized in that** the connecting portions (4, 4a, 4b, 4c) of axially succeeding meandering paths (2, 2a, 2b, 2c) are mutually displaced in the circumferential direction (U).

3. Stent according to claim 2, **characterized in that** the connecting portions (4a, 4a, 4b, 4c) are displaced in the circumferential direction (U) by half a meander period.

4. Stent according to one of the claims 1 to 3, **characterized in that** the meandering paths (2, 2a, 2b, 2c) are constructed in zig-zag form (with tips).

5. Stent according to one of the claims 1 to 3, **characterized in that** the meandering paths have a sinusoidal construction.

6. Stent according to one of the preceding claims, **characterized in that** the meandering paths have an oval construction.

7. Stent according to one of the preceding claims, **characterized in that** facing areas (3, 3a, 3'a, 3b) of the meandering paths (2, 2a, 2b, 2c) are aligned in the axial direction (A).

8. Stent according to one of the preceding claims, **characterized, in that** the width of the connecting areas (4, 4a, 4b, 4c) in the circumferential direction (U) is not greater than the widths of the legs of the meandering paths (2, 2a, 2b, 2c).

## Revendications

1. Extenseur comportant plusieurs rangées en méandre (2, 2a,2b, 2c) s'étendant axialement l'une derrière l'autre sur sa surface latérale U, extenseur dans lequel des zones disposées axialement en regard sont reliées entre elles par des liaisons (4, 4a, 4b, 4c) alors que les suivantes imbriquées orientées dans le sens périmétrique et disposées axialement en regard ne sont pas reliées entre elles **caractérisé en ce que** l'extenseur (1) est réalisé dans une feuille de tôle en alliage à mémoire de forme présentant un état de température basse dans lequel des branches en méandre des rangées en méandre sont jointives et un état de température élevée correspondant à une dilatation radiale et **en ce qu'**il existe à chaque fois dans la même rangée de méandre (2, 2a, 2b, 2c) pour deux liaisons (4, 4a, 4b, 4c) au moins à chaque fois deux zones (3, 3a, 3b, 3c) non reliées entre elles.

2. Extenseur selon la revendication 1, **caractérisé en ce que** les segments de liaison (4,4a,4b,4c) de bandes en méandres (2,2a,2b,2c) se suivant axialement sont disposés décalées les unes par rapport aux autres dans le sens périmétrique (U).

3. Extenseur selon la revendication 2, **caractérisé en ce que** les segments de liaison (4,4a,4b,4c) sont décalés dans le sens périmétrique (U) de la moitié de la période d'ondulation d'un méandre.

4. Extenseur selon l'une quelconque des revendications 1 à 3, **caractérisé en ce que** les bandes en méandres (2,2a,2b,2c) sont conformées en zigzag (avec pointes).

5. Extenseur selon l'une quelconque des revendications 1 à 3, **caractérisé en ce que** les bandes en méandres présentent une forme sinusoïdale.

6. Extenseur selon l'une quelconque des revendications précédentes, **caractérisé en ce que** les bandes en méandres présentent une forme ovale.

7. Extenseur selon l'une quelconque des revendications précédentes, **caractérisé en ce que** des zones (3,3a,3'a,3b) des bandes en méandres (2,2a,2b,2c) sont alignées selon la direction axiale (A).

8. Extenseur selon l'une quelconque des revendications précédentes, **caractérisé en ce que** la largeur des zones de liaison (4,4a,4b,4c) n'est pas supérieure, dans le sens périmétrique (U) à la largeur des branches des bandes en méandres (2,2a,2b,2c).
